(19) Europäisches Patentamt / European Patent Office / Office européen des brevets

(11) **EP 2 796 196 B1**

(12) **FASCICULE DE BREVET EUROPEEN**

(45) Date de publication et mention de la délivrance du brevet:
**19.06.2019 Bulletin 2019/25**

(51) Int Cl.:
**B01J 23/882** $^{(2006.01)}$     **C10G 45/08** $^{(2006.01)}$

(21) Numéro de dépôt: **14305382.5**

(22) Date de dépôt: **18.03.2014**

(54) **ADSORBANT CATALYTIQUE POUR LA CAPTATION DE L'ARSENIC ET L'HYDRODÉSULFURATION SÉLECTIVE DES ESSENCES**

KATALYTISCHES ADSORPTIONSMITTEL ZUR AUFNAHME VON ARSEN, UND SELEKTIVE HYDRODESULFURIERUNG DER ESSENZEN

CATALYTIC ADSORBER FOR ARSENIC COLLECTION AND SELECTIVE HYDRODESULPHURISATION OF GASOLINE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.04.2013 FR 1353828**

(43) Date de publication de la demande:
**29.10.2014 Bulletin 2014/44**

(73) Titulaire: **IFP Energies nouvelles**
**92500 Rueil-Malmaison (FR)**

(72) Inventeurs:
• **Leflaive, Philibert**
  **69780 Mions (FR)**
• **Marion, Marie-Claire**
  **69390 Vernaison (FR)**
• **Daudin, Antoine**
  **69960 Corbas (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A1- 1 892 039     US-A- 4 111 795**

## Description

Domaine de l'invention

La présente invention se rapporte au domaine de l'hydrotraitement des coupes essences, notamment des coupes essences issues des unités de craquage catalytique en lit fluidisé (FCC). Plus particulièrement, la présente invention concerne un adsorbant catalytique ayant les propriétés de capter l'arsenic et simultanément de désulfurer sélectivement dans le temps une charge hydrocarbonée, tout en préservant un indice d'octane élevé. L'invention concerne aussi un procédé d'hydrotraitement mettant en oeuvre ledit adsorbant catalytique.

Les spécifications sur les carburants automobiles prévoient une forte diminution de la teneur en soufre dans ces carburants, et notamment dans les essences. Cette diminution est destinée à limiter, notamment, la teneur en oxyde de soufre et d'azote dans les gaz d'échappement d'automobiles. La législation européenne précise les spécifications des carburants essences. En 2009, elles sont au maximum de 10 ppm poids (parties par millions) de soufre. Pour atteindre ces spécifications, il est nécessaire de traiter les essences par des procédés de désulfuration. Cette teneur pourrait être encore revue à la baisse dans les prochaines années.

Les sources principales de soufre dans les bases pour essences sont les essences dites de craquage, et principalement, la fraction d'essence issue d'un procédé de craquage catalytique d'un résidu de la distillation atmosphérique ou sous vide d'un pétrole brut. La fraction d'essence issue du craquage catalytique, qui représente en moyenne 40% des bases essence, contribue en effet pour plus de 90% à l'apport de soufre dans les essences. Par conséquent, la production d'essences peu soufrées nécessite une étape de désulfuration des essences de craquage catalytique. Parmi les autres sources d'essences pouvant contenir du soufre, citons également les essences de coker, de visbreaker ou, dans une moindre mesure, les essences issues de la distillation atmosphérique ou les essences de vapocraquage.

L'élimination du soufre dans les coupes essences consiste à traiter spécifiquement ces essences riches en soufre par des procédés de désulfuration en présence d'hydrogène. On parle alors de procédés d'hydrodésulfuration (HDS). Cependant, ces coupes essences et plus particulièrement les essences issues du FCC contiennent une part importante de composés insaturés sous forme de mono-oléfines (environ 20 à 50% poids) qui contribuent à un bon indice d'octane, de dioléfines (0,5 à 5% poids) et d'aromatiques. Ces composés insaturés sont instables et réagissent au cours du traitement d'hydrodésulfuration. Les dioléfines forment des gommes par polymérisation lors des traitements d'hydrodésulfuration. Cette formation de gommes entraîne une désactivation progressive des catalyseurs d'hydrodésulfuration ou un bouchage progressif du réacteur. En conséquence, les dioléfines doivent être éliminées par hydrogénation avant tout traitement de ces essences. Les procédés de traitement traditionnels désulfurent les essences de manière non sélective en hydrogénant une grande partie des mono-oléfines, ce qui engendre une forte perte en indice d'octane et une forte consommation d'hydrogène. Les procédés d'hydrodésulfuration les plus récents permettent de désulfurer les essences de craquage riches en mono-oléfines, tout en limitant l'hydrogénation des mono-oléfines et par conséquent la perte d'octane. De tels procédés sont par exemples décrits dans les documents EP-A-1077247 et EP-A-1174485.

Les procédés d'hydrodésulfuration sont opérés de façon ininterrompue sur des durées d'au moins 3 à 5 ans. Les catalyseurs utilisés pour effectuer l'hydrodésulfuration des essences soufrées doivent donc présenter une bonne activité, une bonne sélectivité et une bonne stabilité dans le temps pour être opérés continûment pendant plusieurs années. Or, la présence de métaux lourds tels que le mercure ou l'arsenic, ou de contaminants tels que le phosphore et le silicium sous forme d'organométalliques dans les charges hydrocarbonées à désulfurer entraîne une désactivation rapide des catalyseurs d'hydrotraitement. Il est donc nécessaire d'éliminer ces contaminants de la charge avant de la mettre en contact avec ces catalyseurs d'hydrodésulfuration.

Art Antérieur

Différentes solutions sont proposées pour extraire ces impuretés et plus particulièrement l'arsenic dans les charges hydrocarbonées. En général, un adsorbant est placé soit dans un réacteur situé en amont de l'unité d'hydrodésulfuration soit dans le réacteur d'hydrodésulfuration, en amont du lit catalytique contenant le catalyseur d'hydrodésulfuration. De tels adsorbants sont décrits dans les documents FR2794381 et WO2006/037884. Ces adsorbants sont utilisés en présence d'hydrogène, ce qui présente un inconvénient lorsque les essences à traiter comprennent des composés insaturés. Il en résulte une diminution d'indice d'octane et une baisse de la qualité de l'essence considérée dès l'étape d'adsorption des impuretés. Ces adsorbants présentent également l'inconvénient d'être catalytiquement peu actifs pour les réactions d'hydrodésulfuration. Par ailleurs, Ils occupent un volume non négligeable dans le réacteur, diminuant le volume disponible pour les lits de catalyseurs d'hydrodésulfuration et donc entraînent une perte globale des performances du procédé. Il est donc nécessaire de chercher des solutions qui permettent d'éliminer ces impuretés telles que l'arsenic, avec pour objectif de limiter les réactions d'hydrogénation responsables d'une diminution de l'indice d'octane des essences concernées. Ces solutions doivent aussi permettre d'augmenter les performances d'hydrodésulfuration et sans perte de sélectivité de la réaction d'hydrodésulfuration par rapport à l'hydrogénation des oléfines.

En ce sens, le document EP-A-2072607 décrit un procédé en lit fixe d'adsorption de l'arsenic et de désulfuration d'une charge hydrocarbonée en la mettant en contact avec un adsorbant présentant des propriétés catalytiques désulfurantes. Cet adsorbant permet de capter l'arsenic et de désulfurer une charge hydrocarbonée tout en limitant l'hydrogénation des mono-oléfines en combinant à la fois un élément du groupe VIII et un élément du groupe VIB. Cependant, au cours de son utilisation dans le temps, l'activité d'hydrodésulfuration de cet adsorbant n'est pas suffisante et diminue fortement. On observe une augmentation des réactions d'hydrogénation des mono-oléfines au détriment des réactions d'hydrodésulfuration. L'activité catalytique de cet adsorbant est très vite diminuée.

US 4 111 795 décrit des catalyseurs comprenant entre 18 et 30 % poids de $MoO_3$ et entre 4 et 12 % poids de CoO.

EP1 892 039 décrit des catalyseurs comprenant des éléments des groupes VIB et VIII, dont les teneurs exprimées en oxyde des éléments des groupes VIB et VIII sont respectivement comprises entre 1 et 20 % poids et 0,1 et 20 % poids et qui présentent en outre un rapport molaire élément du groupe VIII/élement du groupe VIB compris entre 0,1 et 0,8.

Ainsi il existe toujours un besoin de disposer d'un solide adsorbant de l'arsenic ayant des propriétés d'adsorption de métaux lourds et des propriétés catalytiques en hydrodésulfuration optimisées, c'est-à-dire présentant un bon compromis entre l'activité en hydrodésulfuration (HDS), et une sélectivité maximale de réactions d'hydrodésulfuration par rapport aux réactions d'hydrogénation des oléfines (HDS/HYD) et dont les propriétés d'adsorption et d'activité catalytique après captation soient stables dans le temps. La présente invention propose un nouvel adsorbant catalytique pour pallier les inconvénients des adsorbants de l'art antérieur.

Résumé et intérêt de l'invention

La présente invention concerne un adsorbant catalytique comprenant au moins du cobalt et du molybdène déposés sur un support poreux dans lequel la teneur en cobalt, exprimée en oxyde CoO, est comprise entre 15 et 25% poids par rapport au poids total dudit adsorbant et la teneur en molybdène, exprimée en oxyde $MoO_3$, est comprise entre 5 et 16% poids par rapport au poids total dudit adsorbant et avec un rapport molaire Co/Mo compris entre 1 et 6.

La demanderesse a constaté, de manière surprenante, qu'un adsorbant catalytique qui comprenait comme phase métallique active du cobalt et du molybdène dont les teneurs répondent aux conditions mentionnées ci-dessus présente non seulement une bonne capacité d'adsorption de l'arsenic mais également une activité en hydrodésulfuration et une sélectivité en faveur de l'hydrodésulfuration par rapport à l'hydrogénation des oléfines qui sont stables dans le temps. La mise en oeuvre de l'adsorbant selon l'invention permet d'éliminer simultanément l'arsenic et le soufre d'une charge hydrocarbonée comprenant également des mono-oléfines, pendant une longue période d'utilisation, tout en limitant sélectivement l'hydrogénation des mono-oléfines contenues dans ladite charge.

Un autre objet de l'invention est un procédé d'hydrotraitement d'une charge hydrocarbonée dans laquelle on met en contact la charge hydrocarbonée en présence d'hydrogène avec l'adsorbant catalytique selon l'invention, en particulier, un procédé d'adsorption de l'arsenic et de désulfuration d'une charge hydrocarbonée comprenant des mono-oléfines, du soufre et de l'arsenic dans lequel on met en contact, ladite charge hydrocarbonée en présence d'hydrogène avec l'adsorbant catalytique selon l'invention pour produire un effluent dont la teneur en soufre est réduite et appauvri en arsenic et sans perte d'indice d'octane.

Par "adsorbant catalytique" aussi appelé "adsorbant" dans la suite du texte, on entend au sens de la présente invention, un solide qui permet de capter par adsorption des impuretés telles que l'arsenic et qui permet simultanément d'éliminer les composés soufrés contenus dans la charge par une réaction catalytique en présence d'hydrogène.

Description détaillée de l'invention

La présente invention concerne un adsorbant catalytique comprenant au moins du cobalt et du molybdène déposés sur un support poreux dans lequel la teneur en cobalt, exprimée en oxyde CoO, est comprise entre 15 et 25% poids par rapport au poids total dudit adsorbant et la teneur en molybdène, exprimée en oxyde $MoO_3$, est comprise entre 5 et 16% poids par rapport au poids total dudit adsorbant et avec un rapport molaire Co/Mo compris entre 1 et 6. Dans une variante selon l'invention, ledit adsorbant catalytique comprend un rapport molaire Co/Mo compris entre 1 et 3,5.

L'adsorbant catalytique selon l'invention comprend avantageusement en outre du phosphore. Dans ce cas, la teneur en phosphore, exprimée en oxyde $P_2O_5$, est comprise entre 0,2 et 8% poids par rapport au poids total dudit adsorbant, de manière préférée comprise entre 0,3 et 5 % poids et de manière plus préférée entre 0,4 et 4% poids.

Les métaux dudit adsorbant catalytique sont déposés sur un support minéral amorphe sélectionné dans le groupe constitué par les alumines, la silice, les silices-alumines, le carbure de silicium, les oxydes de titane seuls ou en mélange avec l'alumine ou de la silice alumine, les oxydes de magnésium seuls ou en mélange avec l'alumine ou de la silice alumine. De préférence le support est sélectionné dans le groupe constitué par les alumines, la silice et les silices-alumines. De manière très préférée, le support est essentiellement constitué par au moins une alumine, c'est-à-dire qu'il comprend au moins 51% poids, de préférence au moins 60% poids, de manière très préféré au moins 80 % poids, voire au moins 90% poids. L'alumine peut être de différentes formes cristallines comme par exemple, de l'alumine alpha, de l'alumine beta, de l'alumine gamma, de l'alumine delta, de l'alumine êta, de l'alumine thêta, de la boehmite ou leurs mélanges. La surface spécifique du catalyseur est comprise entre 50 et 350 m2/g, de préférence entre 70 et 300 m2/g, de manière très préférée entre 90 et 250 m2/g. La surface spécifique est mesurée par la technique BET (norme ASTM D3663) sur le solide à l'état oxyde. La porosité est telle que celui-ci possède un volume poreux compris entre 0,4 et 1,4 cm3/g, de préférence compris entre 0,5 et 1,3 cm3/g. Le volume poreux est mesuré par porosimétrie au mercure selon la norme ASTM D4284-92 avec un angle de mouillage de 140°.

Les métaux sont déposés sur le support de l'adsorbant catalytique selon l'invention selon des techniques bien connues de l'homme du métier, par exemple par imprégnation à partir de solution de précurseurs de métaux. L'imprégnation peut être par exemple réalisée selon le mode connu d'imprégnation à sec dans lequel on introduit la quantité d'éléments désirée sous forme de sels solubles dans le solvant choisi, par exemple de l'eau déminéralisée, de façon à remplir aussi exactement que possible la porosité du support. Le support ainsi rempli par la solution est de préférence séché. Le support préféré est l'alumine qui peut être préparée à partir de tout type de précurseurs et outils de mise en forme connus de l'homme du métier.

Les métaux peuvent être déposés en co-imprégnation ou par ajout successif.

Selon une premier mode de réalisation, on dépose sur le support les métaux cobalt et molybdène en une seule étape, par imprégnation à sec dudit support au moyen d'une solution contenant la quantité désirée de cobalt et molybdène.

Alternativement selon un deuxième mode de réalisation, dans une première étape on dépose par imprégnation le cobalt et ensuite le molybdène ou inversement le molybdène et ensuite le cobalt. Selon un troisième mode de réalisation, on réalise une première étape d'imprégnation sur le support des métaux cobalt et molybdène. Une seconde imprégnation de cobalt ou de molybdène seul est ensuite réalisée, afin d'ajuster le rapport molaire Co/Mo. Dans ce deuxième ou troisième mode de réalisation, avant la deuxième imprégnation, le support imprégné est séché et éventuellement calciné.

Si le catalyseur comprend du phosphore, celui peut être ajouté dans les solutions d'imprégnation ou éventuellement être déposé après dépôt du cobalt et du molybdène.

On utilise avantageusement comme solution d'imprégnation du cobalt une solution contenant du nitrate de cobalt, l'hydroxyde de cobalt ou le carbonate de cobalt, seule ou en mélange. Comme solution d'imprégnation du molybdène, on utilise avantageusement une solution contenant l'heptamolybdate d'ammonium ou l'oxyde de molybdène, seule ou en mélange. Lorsque le phosphore est présent dans l'adsorbant catalytique selon l'invention, on utilise avantageusement comme précurseur l'acide phosphorique en solution d'imprégnation. Cependant tout autre sel connu de l'homme du métier présentant une solubilité suffisante en solution aqueuse et décomposable lors d'une étape de séchage ou tout type de traitement oxydant peut également être utilisé.

Après introduction des métaux et éventuellement du phosphore, l'adsorbant catalytique selon l'invention est préférentiellement soumis à un traitement de calcination. Ce traitement a pour but de transformer les précurseurs moléculaires des métaux en phase oxyde. Il s'agit dans ce cas d'un traitement oxydant mais un simple séchage de l'adsorbant catalytique peut également être effectué. De manière préférée, l'adsorbant catalytique est soumis à un traitement de calcination, préalablement à sa mise en oeuvre dans le procédé d'hydrodésulfuration selon l'invention. Ledit traitement de calcination est avantageusement mis en oeuvre sous air ou sous oxygène dilué, à une température comprise entre 200°C et 550 °C, de préférence entre 300°C et 500°C.

Après calcination, les métaux déposés sur le support se trouvent sous forme d'oxyde. Avantageusement, l'adsorbant catalytique calciné est en outre soumis à un traitement de sulfuration avant sa mise en oeuvre dans le procédé d'hydrodésulfuration selon l'invention. La sulfuration est réalisée en milieu sulforéducteur, c'est à dire en présence d'$H_2S$ et d'hydrogène, afin de transformer les oxydes métalliques en sulfures de métaux de transition tels que le $MoS_2$, le $Ni_3S_2$ et le $Co_9S_8$. La sulfuration est réalisée en injectant sur l'adsorbant catalytique un flux contenant de l'$H_2S$ et de l'hydrogène, ou bien un composé soufré susceptible de se décomposer en $H_2S$ en présence de l'adsorbant catalytique et de l'hydrogène. Les polysulfures tel que le diméthyldisulfure sont des précurseurs d'$H_2S$ couramment utilisés pour sulfurer les catalyseurs. La température est ajustée afin que l'$H_2S$ réagisse avec les oxydes métalliques pour former des sulfures métalliques. Cette sulfuration peut être réalisée in situ ou ex situ (en dedans ou en dehors du réacteur d'hydrodésulfuration) à des températures comprises entre 200 et 600°C et plus préférentiellement entre 300 et 500°C. Pour être actifs, les métaux doivent être substantiellement sulfurés. Un élément est considéré comme substantiellement sulfuré lorsque le rapport molaire entre le soufre (S) présent sur l'adsorbant catalytique et le dit élément est au moins égal à 60% du rapport molaire théorique correspondant à la sulfuration totale de l'élément considéré :

$$S/\text{élément})_{\text{adsorbant catalytique}} >= 0{,}6 \times (S/\text{élément})_{\text{théorique}}$$

avec :

- (S/élément)$_{adsorbant\ catalytique}$ rapport molaire entre le soufre (S) et l'élément présent sur l'adsorbant catalytique.
- (S/élément)$_{théorique}$ rapport molaire entre le soufre et l'élément correspondant à la sulfuration totale de l'élément en sulfure.

Ce rapport molaire théorique varie selon l'élément considéré :

- (S/Co)$_{théorique}$ = 8/9
- (S/Mo)$_{théorique}$ =2/1

Le rapport molaire entre le soufre présent sur l'adsorbant et l'ensemble des éléments est de préférence au moins égal à 60% du rapport molaire théorique correspondant à la sulfuration totale de chaque élément en sulfure, le calcul étant effectué au prorata des fractions molaires relatives de chaque élément.

Après sulfuration, l'adsorbant catalytique selon l'invention est prêt pour être utilisé dans un procédé d'adsorption d'arsenic et de d'hydrodésulfuration d'une charge hydrocarbonée comprenant des composés insaturés.

Un autre objet de l'invention est un procédé d'hydrotraitement d'une charge hydrocarbonée dans laquelle on met en contact la charge hydrocarbonée en présence d'hydrogène avec l'adsorbant catalytique selon l'invention. Dans le sens de la présente invention, le procédé d'hydrotraitement selon l'invention est un procédé d'adsorption de l'arsenic et éventuellement du silicium et de désulfuration au moins partielle de la charge hydrocarbonée en présence d'hydrogène pour produire un effluent à teneur en soufre réduite et appauvri en métaux lourds en particulier l'arsenic, avec une perte limitée de l'indice d'octane.

Le procédé d'hydrotraitement selon l'invention permet d'éliminer l'arsenic et de transformer simultanément une partie des composés soufrés organiques en H$_2$S. Il permet également de limiter le taux d'hydrogénation des mono-oléfines et dioléfines. Le taux d'hydrogénation des oléfines est avantageusement inférieur à 50%, préférentiellement inférieur à 30%, et de manière encore préférée inférieur à 20%.

La charge hydrocarbonée à traiter est une essence de craquage catalytique issue d'unités de craquage catalytique, de craquage thermique ou de vapocraquage. Le procédé peut également s'appliquer au traitement de mélanges d'essences de distillation directe qui peuvent contenir des métaux lourds issus du brut avec des essences de craquage comprenant des mono-oléfines et des dioléfines. De manière préférée, la charge hydrocarbonée à traiter est une essence de craquage catalytique comprenant entre 5 % et 60% poids de mono-oléfines, entre 50 ppm et 6000 ppm poids de composés soufrés et entre 10 et 1000 ppb poids d'arsenic.

Les composés soufrés contenus dans la charge hydrocarbonée à traiter peuvent être des composés soufrés organiques tels que par exemple, les mercaptans, les composés thiophéniques, benzothiophèniques et autres composés soufrés aromatiques, les composés disulfites, etc.

Les composés arséniés contenus dans la charge hydrocarbonée à traiter peuvent être des composés arséniés organiques tels que par exemple la triméthylarsine ou la triéthylarsine.

Les mono-oléfines désignent des molécules hydrocarbonées présentant une unique double liaison carbone-carbone et les dioléfines sont des molécules hydrocarbonées comprenant au moins deux doubles liaisons carbone-carbone. Les mono-oléfines et les dioléfines peuvent être des molécules hydrocarbonées linéaires, ramifiées et/ou cycliques.

L'adsorbant catalytique selon l'invention est mis en oeuvre avantageusement dans des conditions opératoires telles que la vitesse d'adsorption de l'arsenic soit maximisée, tout en limitant la vitesse d'hydrogénation des oléfines.

La mise en contact s'effectue généralement à une température comprise entre 200 et 400°C, à une pression comprise entre 0,2 et 5 MPa et avec un rapport du débit d'hydrogène sur le débit de charge hydrocarbonée compris entre 50 et 800 Nm$^3$/m$^3$.

Avantageusement, l'adsorbant catalytique est soumis à un traitement de calcination préalablement à sa mise en oeuvre. De préférence selon l'invention, l'adsorbant catalytique calciné est en outre soumis à un traitement de sulfuration avant sa mise en oeuvre. Avantageusement, ladite sulfuration est réalisée de telle sorte que le taux de sulfuration des métaux constituants ledit adsorbant catalytique est d'au moins 60%.

L'hydrogène utilisé peut être issu de toute source d'hydrogène. De préférence on utilise de l'hydrogène frais issu de la raffinerie et/ou de l'hydrogène recyclé d'une unité d'hydrodésulfuration, de préférence de l'unité d'hydrodésulfuration de la coupe hydrocarbonée à purifier.

Plusieurs technologies de réacteur sont envisageables pour réaliser l'adsorption et l'hydrodésulfuration d'une charge hydrocarbonée en présence de l'adsorbant catalytique selon l'invention. La technologie la plus classique et la plus répandue étant la technologie en lit fixe. Dans ce cas, un réacteur est chargé avec l'adsorbant catalytique selon l'invention, fonctionnant en adsorption de l'arsenic et en hydrodésulfuration, en principe jusqu'à l'apparition d'arsenic dans l'effluent de sortie (phénomène connu de l'homme du métier sous le vocable de perçage). Dans certains cas la quantité totale de l'adsorbant empoisonné peut être remplacée par une quantité équivalente d'adsorbant frais. Le choix d'une technologie de remplacement de l'adsorbant catalytique selon l'invention n'est pas considéré dans le cadre de la présente invention comme un élément limitatif. L'adsorbant catalytique peut être mis en oeuvre dans un réacteur à lit mobile, c'est à dire que l'adsorbant usé est soutiré en continu, et remplacé par de l'adsorbant catalytique frais. Ce type de technologie permet de maintenir la captation de l'arsenic par l'adsorbant catalytique et d'éviter le perçage de ce dernier dans les effluents produits. Parmi d'autres solutions, citons la mise en oeuvre des réacteurs en lit expansé qui permet également un soutirage et un appoint continu d'adsorbant catalytique afin de maintenir l'activité d'hydrodésulfuration de l'adsorbant catalytique.

Le procédé d'hydrotraitement selon l'invention est de préférence couplé avec au moins une étape d'hydrodésulfuration catalytique ou d'hydrogénation sélective complémentaire qui est effectuée sur l'effluent issu de la mise en contact avec l'adsorbant catalytique selon l'invention. Ainsi l'étape de traitement de la charge hydrocarbonée par l'adsorbant est considéré comme un prétraitement qui permet notamment de préserver l'activité catalytique du catalyseur utilisé dans l'étape d'hydrodésulfuration ou d'hydrogénation sélective subséquente.

Ainsi le procédé d'hydrotraitement selon l'invention, comprend une ou plusieurs autres étapes complémentaires d'hydrodésulfuration ou d'hydrogénation sélective dans la(s)quelle(s) on met en contact l'effluent issu de la mise en contact de la charge hydrocarbonée avec l'adsorbant catalytique selon l'invention, avec au moins un autre catalyseur d'hydrodésulfuration ou d'hydrogénation sélective des dioléfines présentes dans la charge en oléfines. La(es)dite(s) étapes complémentaires d'hydrodésulfuration permet(tent) d'éliminer les composés soufrés résiduels contenus dans l'effluent appauvri en arsenic et à plus basse teneur en soufre. Certains de ces composés soufrés résiduels peuvent être issus de l'addition de H$_2$S sur les oléfines présentes dans la charge. L'H$_2$S peut se former au cours de la mise en contact de la charge hydrocarbonée avec l'adsorbant catalytique, c'est-à-dire, pendant l'adsorption de l'arsenic et la désulfuration mettant en oeuvre l'adsorbant catalytique selon l'invention.

La(es)dite(s) étapes complémentaires d'hydrodésulfuration est (sont) mise(s) en oeuvre lorsque l'effluent issu de la mise en contact de la charge hydrocarbonée avec l'adsorbant catalytique présente généralement une teneur en soufre supérieure à 10 ppm et qu'il est nécessaire de produire des essences à faible teneur en soufre répondant aux spécifications actuelles qui sont dans de nombreux pays inférieures à 10 ppm. L'effluent débarrassé de l'arsenic et d'une partie des composés soufrés est alors traité dans au moins une desdites étapes complémentaires d'hydrodésulfuration sélective. Dans la(es)dite(s) étapes, ledit effluent est mis en contact avec au moins un autre catalyseur d'hydrodésulfuration dans des conditions opératoires qui peuvent être identiques ou différentes de celles de la mise en contact de la charge hydrocarbonée avec l'adsorbant catalytique.

Le(les) catalyseur(s) mis en oeuvre dans la(es)dite(s) étapes complémentaires d'hydrodésulfuration est (sont) protégé(s) de la désactivation par l'arsenic présent dans la charge grâce à l'adsorbant catalytique selon l'invention. Ainsi des catalyseurs d'hydrodésulfuration très sélectifs qui sont sensibles à la présence d'arsenic peuvent être mis en oeuvre dans la(es)dite(s) étape(s) complémentaires d'hydrodésulfuration. Tout catalyseur d'hydrodésulfuration peut être utilisé dans la(es)dite(s) étape(s) complémentaire(s) d'hydrodésulfuration.

De préférence, on utilise des catalyseurs présentant une sélectivité élevée vis-à-vis des réactions d'hydrodésulfuration par rapport aux réactions d'hydrogénation des oléfines. De tels catalyseurs comprennent au moins un support minéral amorphe et poreux, un métal du groupe VIB, un métal du groupe VIII. Le métal du groupe VIB est préférentiellement le molybdène ou le tungstène et le métal du groupe VIII est préférentiellement le nickel ou le cobalt. Le support est généralement sélectionné dans le groupe constitué par les alumines, la silice, les silice-alumines, le carbure de silicium, les oxydes de titane seuls ou en mélange avec de l'alumine ou de la silice alumine, les oxydes de magnésium seuls ou en mélange avec de l'alumine ou de la silice alumine. De préférence, le support est sélectionné dans le groupe constitué par les alumines, la silice et les silice-alumines. De préférence le catalyseur d'hydrodésulfuration utilisé dans le ou les étapes complémentaires d'hydrodésulfuration présente les caractéristiques suivantes:

- la teneur en éléments du groupe VIB est comprise (bornes incluses) entre 1 et 20% poids d'oxydes d'éléments du groupe VIB;
- la teneur en éléments du groupe VIII est comprise (bornes incluses) entre 0,1 et 20% poids d'oxydes d'éléments du groupe VIII;
- le rapport molaire (éléments du groupe VIII / éléments du groupe VIB) est compris (bornes incluses) entre 0,1 et 0,8.

Un catalyseur d'hydrodésulfuration très préféré comprend du cobalt et du molybdène et a les caractéristiques mentionnées ci-dessus.

Par ailleurs le catalyseur d'hydrodésulfuration peut comprendre du phosphore. Dans ce cas, la teneur en phosphore est de préférence comprise (bornes incluses) entre 0,1 et 10% poids de P$_2$O$_5$ par rapport au poids total de catalyseur et le rapport molaire phosphore sur éléments du groupe VIB est supérieur ou égal a 0,25, de préférence supérieur ou égal a 0,27.

Dans la(es)dite(s) étape(s) complémentaire(s) d'hydrodésulfuration, l'effluent à plus basse teneur en soufre et appauvri en arsenic issu la mise en contact de la charge hydrocarbonée avec l'adsorbant catalytique selon l'invention est mis en contact avec au moins un autre catalyseur d'hydrodésulfuration sélective dans les conditions opératoires suivantes :

- une température comprise entre environ 210 et environ 410°C, préférentiellement entre 240 et 360°C;
- une pression totale comprise entre 0,2 et 5 MPa et plus préférentiellement entre 0,5 et environ 3 MPa;
- un ratio volume d'hydrogène par volume de charge hydrocarbonée, compris entre 50 et 800 $Nm^3/m^3$ et plus préférentiellement entre 60 et 600 $Nm^3/m^3$.

Dans une variante du procédé selon l'invention, les conditions opératoires de la mise en contact de la charge hydrocarbonée avec l'adsorbant catalytique selon l'invention sont identiques à celles mises en oeuvre dans la(es)dite(es) étape(s) complémentaire(s) d'hydrodésulfuration.

Selon une autre forme de réalisation, l'étape d'hydrotraitement de l'effluent issu de l'étape d'adsorption au moyen de la masse d'adsorption selon l'invention est une hydrogénation sélective qui permet l'hydrogénation des dioléfines en oléfines et éventuellement des composés soufrés insaturés mais également la transformation (alourdissement) des composés soufrés légers (i.e. ayant une température inférieure à celle du thiophène) en des composés soufrés dont la température est supérieure à celle du thiophène, par exemple par addition des mercaptans sur des oléfines.

Cette étape d'hydrogénation est effectuée en présence d'hydrogène et d'un catalyseur contenant au moins un métal du groupe VIb et au moins un métal non noble du groupe VIII déposés sur un support poreux. De préférence on utilise un catalyseur dont:

- la teneur en poids d'oxyde de l'élément du groupe VIb est comprise entre 6 et 18% par rapport au poids du catalyseur;
- la teneur en poids d'oxyde de l'élément du groupe VIII est comprise entre 4 et 12% par rapport au poids du catalyseur;
- la surface spécifique du catalyseur est comprise entre 200 et 270 $m^2/g$;
- la densité de l'élément du groupe VIb, exprimée comme étant le rapport entre ladite teneur en poids d'oxyde de l'élément du groupe VIb et la surface spécifique du catalyseur, est comprise entre 4 et $6.10^{-4}$ $g/m^2$;
- le rapport molaire entre le métal du groupe VIII et le métal du groupe VIb est compris 0,6 et 3 mol/mol.

Le métal du groupe VIb est de préférence choisi parmi le molybdène et le tungstène, de manière très préférée le métal du groupe VIb est le molybdène.

Le métal du groupe VIII est de préférence le nickel et/ou le cobalt, de manière très préférée le nickel.

L'hydrogène est introduit généralement en faible excès, jusqu'a 5 moles par mole, par rapport à la stoechiométrie, nécessaire pour hydrogéner les dioléfines (une mole d'hydrogène par mole de dioléfine). Le mélange constitué de l'essence et d'hydrogène est mis en contact avec le catalyseur sous une pression comprise entre 0,5 et 5 MPa, une température comprise entre 80 et 220°C, avec une vitesse spatiale liquide (LHSV) comprise entre 1 et 10 $h^{-1}$, la vitesse spatiale liquide étant exprimée en litre de charge par litre de catalyseur et par heure (L/L.h).

Dans une variante du procédé selon l'invention, l'adsorbant catalytique selon l'invention peut être placé en position de lit de garde d'un ou plusieurs réacteur(s) contenant le(s) catalyseur(s) mis en oeuvre dans la(es)dite(es) étape(s) complémentaire(s) d'hydrodésulfuration et/ou d'hydrogénation sélective.

Dans une autre variante du procédé selon l'invention, l'adsorbant catalytique selon l'invention est placé dans un réacteur dit d'adsorption. Ce réacteur est séparé et est placé en amont du (des) réacteur(s) contenant le(s) catalyseur(s) mis en oeuvre dans la(es)dite(es) étape(s) complémentaire(s) d'hydrodésulfuration et/ou d'hydrogénation sélective.

Dans toutes les variantes du procédé selon l'invention, mettant en oeuvre au moins une étape complémentaire d'hydrodésulfuration et/ou d'hydrogénation sélective, le rapport de volume de l'adsorbant catalytique selon l'invention par rapport au volume de (des) catalyseur(s) mis en oeuvre dans la(es)dite(es) étape(s) complémentaire(s) d'hydrodésulfuration et/ou d'hydrogénation sélective est avantageusement compris entre 4 et 50%, de préférence entre 5 et 40%, de manière plus préférée entre 5 et 35%.

Exemples

Les exemples qui suivent illustrent l'invention sans pour autant en limiter la portée.

Exemple 1 : Préparation des solides adsorbants

Trois adsorbants sont préparés par imprégnation à sec d'un support alumine à partir d'une solution aqueuse contenant les précurseurs de cobalt et de molybdène. Ces derniers sont le nitrate de cobalt et l'heptamolybdate d'ammonium pour les solides A et B. Dans le cas du solide C, les précurseurs sont l'hydroxyde de cobalt, l'oxyde de molybdène et l'acide phosphorique. A l'issue de l'étape d'imprégnation, les solides sont laissés à maturer pendant 2 heures puis séchés à l'étuve sous vide à 90°C durant une nuit, puis calcinés à 450°C pendant 6 heures.

A l'issue de leur préparation, les teneurs en Co, Mo et P sont déterminées par fluorescence X. Les résultats, exprimés en % poids d'oxyde CoO, $MoO_3$ et $P_2O_5$ sont donnés dans le tableau 1 ci-dessous :

Tableau 1 : Caractéristiques des solides

| Solide | A (comparatif) | B (selon l'invention) | C (selon l'invention) |
|---|---|---|---|
| CoO (% poids) | 2.3 | 20 | 20 |
| $MoO_3$ (% poids) | 8 | 8 | 10 |
| $P_2O_5$ (% poids) | - | - | 1.4 |
| Co/Mo (rapport atomique) | 0.3 | 2.9 | 2.4 |

Exemple 2 : Évaluation des performances catalytiques et de captation de l'arsenic

On procède ensuite à l'évaluation de ces solides pour leur performances en hydrodésulfuration d'une essence issue du FCC (coupe 50-245°C) contenant 360 ppm poids de soufre. Afin d'évaluer les performances des solides pour la captation d'arsenic, une partie de cette charge est dopée par un composé arsénié (triphényl arsine) de façon à atteindre une concentration de 3000 ppb poids d'arsenic. Pour réaliser ces essais, on utilise une unité pilote équipée d'un réacteur tubulaire à lit fixe traversé.

La quantité d'arsenic dans la charge et dans les effluents est mesurée par absorption atomique, le soufre dans la charge et dans les effluents est déterminé par fluorescence X et les oléfines dans la charge et dans les effluents sont analysées par chromatographie en phase gaz. Ces grandeurs permettent ainsi de calculer les rendements en hydrodésulfuration (HDS), en hydrogénation des oléfines (HydO) et en captation d'arsenic (HDAs).

Les solides adsorbants sont évalués dans les mêmes conditions, détaillées ci-dessous. Pour conduire les réactions, 20 ml de solides adsorbants sont introduits dans le réacteur et sont sulfurés à 350°C sous un mélange gazeux ($H_2/H_2S$) à (85/15) % vol./vol.

Dans une première phase, l'essence de FCC est d'abord injectée en présence d'$H_2$ (rapport $H_2$/charge = 300 Nl/l) et les performances en HDS, HydO et HDAs sont suivies au cours du temps à 250°C et sous un pression totale de 2 MPa. Cette première phase est conduite avec une essence de FCC sans arsenic avec une VVH de 4 $h^{-1}$ pour déterminer les performances en HDS et HydO initiales des solides adsorbants. La durée de cette première phase est fixée 150 heures.

On démarre ensuite la seconde phase qui consiste à traiter l'essence de FCC dans laquelle on a introduit de l'arsenic (composé ajouté = triphényl arsine). Les conditions de température et de pression sont identiques à la première phase à l'exception de la VVH qui est augmentée à 10 $h^{-1}$.

Les résultats obtenus pour les trois solides A, B et C sont présentés dans le tableau 2 ci-dessous. Le temps de perçage indiqué correspond au temps au bout duquel la teneur en arsenic dans l'effluent est supérieure ou égale à 1% de la teneur en arsenic dans la charge ($C/C_0 \geq 1$ %m/m).

Tableau 2 : Performances catalytiques des solides

| | Solide A (comparatif) | Solide B (selon l'invention) | Solide C (selon l'invention) |
|---|---|---|---|
| Rendements HDS et HydO après 150h à VVH 10 $h^{-1}$ :<br>- HDS (% poids)<br>- HydO (% poids) | 28,6<br>2,0 | 27,0<br>2,2 | 26,5<br>1,8 |
| Rendement HDAs : Temps de perçage (h) | 24 | 680 | 690 |
| Rendements après 700h et à VVH 10 $h^{-1}$<br>- HDAs (% poids)<br>- HDS (% poids)<br>- HydO (% poids) | 88.2<br>10<br>2.4 | 99.7<br>22<br>2.2 | 99.6<br>21<br>2.1 |

On observe que le solides A (non-conforme à l'invention) et les solides B, C (conformes à l'invention) présentent des performances comparables en terme d'hydrodésulfuration et d'hydrogénation des oléfines avant l'injection d'arsenic dans l'essence à traiter.

En revanche les solides adsorbants B et C selon l'invention ont une meilleure capacité en adsorption de l'arsenic que le solide A; ceci se traduit par un temps avant perçage multiplié par environ 28 pour les solides adsorbants B et C par rapport au solide A.

On constate également que les solides adsorbants B et C conservent encore une bonne activité en HDS par rapport au solide adsorbant A après 700 heures d'utilisation. Ainsi on observe une baisse de rendement en HDS de 18,5% et 20,8% pour les solides adsorbants B et C respectivement tandis que pour le solide A, cette baisse est 65% après 700 heures. Enfin on remarque que les solide adsorbants B et C, après 700 heures, présentent encore une faible activité en hydrogénation des oléfines.

## Revendications

1. Adsorbant catalytique comprenant au moins du cobalt et du molybdène déposés sur un support poreux dans lequel la teneur en cobalt, exprimée en oxyde CoO, est comprise entre 15 et 25% poids par rapport au poids total dudit adsorbant et la teneur en molybdène, exprimée en oxyde $MoO_3$, est comprise entre 5 et 16% poids par rapport au poids total dudit adsorbant et avec un rapport molaire Co/Mo compris entre 1 et 6.

2. Adsorbant selon la revendication 1 comprenant en outre du phosphore.

3. Adsorbant selon la revendication 2, dans lequel la teneur en phosphore, exprimée en oxyde $P_2O_5$ est comprise entre 0,2 et 8% poids par rapport au poids total dudit adsorbant.

4. Procédé d'hydrotraitement d'une charge hydrocarbonée comprenant au moins une étape d'adsorption catalytique dans lequel on met en contact la charge hydrocarbonée en présence d'hydrogène avec l'adsorbant catalytique selon l'une des revendications 1 à 3.

5. Procédé selon la revendication 4, dans lequel l'adsorbant catalytique est soumis à un traitement de calcination préalablement à sa mise en oeuvre.

6. Procédé selon la revendication 5, dans lequel l'adsorbant catalytique calciné est en outre soumis à un traitement de sulfuration avant sa mise en oeuvre.

7. Procédé selon la revendication 6, dans lequel la sulfuration est réalisée de telle sorte que le taux de sulfuration des métaux constituants ledit adsorbant catalytique est d'au moins 60%.

8. Procédé selon l'une des revendications 4 à 7, dans lequel la mise en contact s'effectue à une température opératoire comprise entre 200 et 400°C, une pression opératoire comprise entre 0,2 et 5 MPa et un rapport du débit d'hydrogène sur le débit de charge hydrocarbonée compris entre 50 et 800 $Nm^3/m^3$.

9. Procédé selon l'une des revendications 4 à 8, dans lequel l'effluent issu de la mise en contact avec l'adsorbant est mis en contact avec au moins un catalyseur d'hydrodésulfuration ou d'hydrogénation sélective.

10. Procédé selon la revendication 9, dans lequel la mise en contact avec le catalyseur d'hydrodésulfuration ou le catalyseur d'hydrogénation sélective est réalisée dans un même réacteur contenant l'adsorbant.

11. Procédé selon les revendications 9 ou 10, dans lequel le rapport de volume dudit absorbant catalytique par rapport au volume dudit catalyseur d'hydrodésulfuration ou d'hydrogénation sélective est compris entre 4 et 50%.

12. Procédé selon l'une quelconque des revendications 4 à 11, dans lequel la charge hydrocarbonée est une essence de craquage catalytique contenant entre 5 et 60% poids de mono-oléfines, entre 50 et 6000 ppm poids de composés soufrés et entre 10 et 1000 ppb poids d'arsenic.

## Patentansprüche

1. Katalytisches Adsorbens, umfassend mindestens Kobalt und Molybdän, die auf einen porösen Träger aufgebracht sind, in dem der Kobaltgehalt, ausgedrückt in Oxid CoO, zwischen 15 und 25 Gew.-% bezogen auf das Gesamtgewicht des Adsorbens beträgt, und der Molybdängehalt, ausgedrückt in Oxid $MoO_3$, zwischen 5 und 16 Gew.-%

bezogen auf das Gesamtgewicht des Adsorbens beträgt, wobei das Molverhältnis Co/Mo zwischen 1 und 6 beträgt.

2. Adsorbens nach Anspruch 1, ferner umfassend Phosphor.

3. Adsorbens nach Anspruch 2, bei dem der Phosphorgehalt, ausgedrückt in Oxid $P_2O_5$, zwischen 0,2 und 8 Gew.-% bezogen auf das Gesamtgewicht des Adsorbens beträgt.

4. Verfahren zur Hydrobehandlung einer Kohlenwasserstoffcharge, umfassend mindestens einen Schritt der katalytischen Adsorption, bei dem die Kohlenwasserstoffcharge im Beisein von Wasserstoff mit dem katalytischen Adsorbens nach einem der Ansprüche 1 bis 3 in Kontakt gebracht wird.

5. Verfahren nach Anspruch 4, bei dem das katalytische Adsorbens einer Kalzinierungsbehandlung vor seinem Einsatz unterzogen wird.

6. Verfahren nach Anspruch 5, bei dem das kalzinierte katalytische Adsorbens ferner einer Sulfidierungsbehandlung vor seinem Einsatz unterzogen wird.

7. Verfahren nach Anspruch 6, bei dem die Sulfidierung derart erfolgt, dass die Sulfidierungsrate der Metalle, die das katalytische Adsorbens bilden, mindestens 60 % beträgt.

8. Verfahren nach einem der Ansprüche 4 bis 7, bei dem das Inkontaktbringen bei einer Betriebstemperatur zwischen 200 und 400 °C, einem Betriebsdruck zwischen 0,2 und 5 MPa und einem Verhältnis der Wasserstoffmenge zur Menge der Kohlenwasserstoffcharge zwischen 50 und 800 $Nm^3/m^3$ erfolgt.

9. Verfahren nach einem der Ansprüche 4 bis 8, bei dem der aus dem Inkontaktbringen mit dem Adsorbens stammende Abfluss mit mindestens einem Katalysator zur selektiven Hydrodesulfurierung oder Hydrierung in Kontakt gebracht wird.

10. Verfahren nach Anspruch 9, bei dem das Inkontaktbringen mit dem Katalysator zur Hydrodesulfurierung oder dem Katalysator zur Hydrierung in einem selben Reaktor, der das Adsorbens enthält, durchgeführt wird.

11. Verfahren nach den Ansprüchen 9 oder 10, bei dem das Volumenverhältnis des katalytischen Adsorbens bezogen auf das Volumen des Katalysators zur selektiven Hydrodesulfurierung oder Hydrierung zwischen 4 und 50 % beträgt.

12. Verfahren nach einem der Ansprüche 4 bis 11, bei dem die Kohlenwasserstoffcharge ein katalytisches Crackbenzin ist, das zwischen 5 und 60 Gew.-% Monoolefine, zwischen 50 und 6000 Gew.-ppm Schwefelverbindungen und zwischen 10 und 1000 Gew.-ppb Arsen enthält.

**Claims**

1. Catalytic adsorbent comprising at least cobalt and molydenum deposited on a porous substrate in which the content of cobalt, expressed in terms of CoO oxide, is between 15 and 25% by weight relative to the total weight of said adsorbent, and the content of molybdenum, expressed in terms of $MoO_3$ oxide, is between 5 and 16 % by weight relative to the total weight of said adsorbent and in which the Co/Mo molar ratio is between 1 and 6.

2. Adsorbent according to claim 1, also comprising phosphorus.

3. Adsorbent according to Claim 2, in which the phosphorus content, expressed in terms of $P_2O_5$ oxide, is between 0.2 and 8% by weight relative to the total weight of said adsorbent.

4. Process for hydrotreatment of a hydrocarbon-containing feedstock comprising at least one catalytic adsorption stage in which the hydrocarbon-containing feedstock is brought into contact, in the presence of hydrogen, with the catalytic adsorbent according to one of Claims 1 to 3.

5. Process according to Claim 4, in which the catalytic adsorbent is subjected to a calcination treatment prior to its use.

6. Process according to Claim 5, in which the calcined catalytic adsorbent is also subjected to a sulfurization treatment before its use.

7.  Process according to Claim 6, in which the sulfurization is carried out in such a way that the sulfurization rate of the metals constituting said catalytic adsorbent is at least 60%.

8.  Process according to one of Claims 4 to 7, in which the contact is made at an operating temperature of between 200 and 400°C, an operating pressure of between 0.2 and 5 MPa, and a ratio of the hydrogen flow rate to the flow rate of the hydrocarbon-containing feedstock of between 50 and 800 Nm3/m3.

9.  Process according to one of Claims 4 to 8, in which the effluent that is obtained from being brought into contact with the adsorbent is brought into contact with at least one catalyst for hydrodesulfurization or selective hydrogenation.

10. Process according to Claim 9, in which the contact with the catalyst for hydrodesulfurization or the catalyst for selective hydrogenation is made in the same reactor that contains adsorbent.

11. Process according to Claim 9 or 10, in which the ratio of the volume of said catalytic absorbent relative to the volume of said catalyst for hydrodesulfurization or selective hydrogenation is between 4 and 50%.

12. Process according to any of Claims 4 to 11, in which the hydrocarbon-containing feedstock is a catalytic cracking gasoline containing between 5 and 60% by weight of monoolefins, between 50 and 6,000 ppm by weight of sulfur-containing compounds, and between 10 and 1,000 ppb by weight of arsenic.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1077247 A
- EP 1174485 A
- FR 2794381
- WO 2006037884 A
- EP 2072607 A
- US 4111795 A
- EP 1892039 A